(19) **Europäisches Patentamt**

**European Patent Office**

**Office européen des brevets**

(11) **EP 0 817 544 B1**

(12) **EUROPEAN PATENT SPECIFICATION**

(45) Date of publication and mention
of the grant of the patent:
**17.03.2004   Bulletin 2004/12**

(51) Int Cl.[7]: **H05B 41/34**, H05B 41/32,
H05B 39/00, H01S 3/00,
H01S 3/092, H01S 3/09,
G01J 3/50, H05H 1/00

(21) Application number: **97304777.2**

(22) Date of filing: **01.07.1997**

(54) **Irradiating device**

Strahlungseinrichtung

Dispositif d'irradiation

(84) Designated Contracting States:
**AT CH DE ES FR GB IT LI SE**

(30) Priority:  **01.07.1996  GB 9613733**

(43) Date of publication of application:
**07.01.1998   Bulletin 1998/02**

(73) Proprietor: **Spectron Laser Systems Limited
Rugby, Warwickshire CV21 1PB (GB)**

(72) Inventors:
• **Greenwood, Mark
Yelvertoft, Northants NN6 6LF (GB)**

• **Sarkies, Paul H.
Rugby, Warwickshire CV22 7RP (GB)**
• **Miller, Donald W.
Rugby, Warwickshire CV22 6HP (GB)**

(74) Representative: **Williamson, Brian et al
Hepworth Lawrence Bryer & Bizley
Merlin House
Falconry Court
Baker's Lane
Epping Essex CM16 5DQ (GB)**

(56) References cited:
**EP-A- 0 215 399          DE-A- 3 439 005
US-A- 5 255 277**

**Description**

This invention relates to an irradiating device and in particular but not exclusively to a method of operating a medical irradiating device producing a pulse of radiant energy of variably controllable energy and pulse length for use in skin treatments such as vein removal or depilation.

It is known to provide light pulses for skin treatment by means of an irradiating device comprising a flash lamp or comprising a laser optically pumped by a flash lamp, the light pulse being delivered to a localised area of skin to effect treatment. It is important to control the output energy and output pulse length associated with the light pulse at the localised area of skin in order to optimise treatment and avoid tissue damage and to adapt the output energy and output pulse length to suit particular skin types which may for example vary according to pigmentation.

US-A-5059192 discloses a method of depilation in which the output energy is controlled in order to determine the depth of penetration of laser light and the output pulse length is controlled to be shorter than the thermal relaxation time of melanin associated with a hair follicle.

It is also known from US-A-5405368 to provide a treatment device in which the irradiating device consists of a flash lamp delivering a pulse of incoherent light and a delivery device for collecting and delivering the light to a treatment area of skin, energy supplied to the light source being provided by a pulse forming circuit to enable the output pulse length of the light source to be variable. Such irradiating devices, whether a flash lamp or laser pumped by a flash lamp, require a relatively high current of the order of 1000 amps to be delivered to the flash lamp during an input pulse length typically in the range 100 μsec to 100 milsec from a capacitive storage device. Variation in the input energy delivered to the input of the irradiating device and shaping of the input pulse of current to effectively vary the input pulse length have hitherto been achieved by switching circuits in which successive passive networks of inductors and capacitors are connected to the input of the light source via a switch which, when triggered, allows total discharge of the energy stored in the capacitors to be discharged as input energy with the input pulse of current decaying to zero according to the time constant of the selected network.

DE3439005 discloses an irradiating system comprising an irradiating device having an input connected to a capacitive energy storage device via a switching means and a control unit connected to the switching means and operable to actuate the switching means.

The present invention seeks to provide an improved method of operating such an irradiating device, particularly with a view to improving the control of input energy and input pulse length to thereby achieve better control of output energy and output pulse length.

According to the present invention, there is disclosed a method of operating an irradiating device to provide a discharge therefrom in the form of a pulse ($E_D$, $t_D$) of radiant energy, the irradiating device having an input selectively coupled to a capacitive energy storage device via a controllable switching means, the method characterised by:

charging the capacitive energy storage device to a voltage $V_S$, wherein a total stored energy in the capacitive energy storage device is between three and four times a maximum energy required to be delivered at each pulse;

closing the switching means to initiate a supply of current (i), associated with discharge of energy from the capacitive energy storage device, to the input of the irradiating device to cause generation of the pulse of radiant energy ($E_D$, $t_D$); and

opening the switching means to terminate the pulse of radiant energy ($E_D$, $t_D$) such that the supply of current to the input of the irradiating device is terminated before complete discharge of the capacitive energy storage device.

There is also provided an irradiating system having an irradating device comprising an input selectively coupled, via a controllable switch, to a capacitive energy storage device connectable, in use, to a voltage source $V_S$, the controllable switch operationally closeable to initiate a supply of current (i), associated with discharge of energy from the capacitive energy storage device, to the input of the irradiating device to cause generation of a pulse ($E_D$, $t_D$) of radiant energy having an energy up to a maximum energy; the irradiating system characterised in that:

the controllable switch is operable to terminate the pulse of radiant energy by terminating the supply of current to the input of the irradiating device at a point before complete discharge of the capacitive energy storage device and a total stored energy in the capacitive energy storage device is between three and four times the maximum energy required to be delivered at each pulse.

An advantage of such a method is that energy storage is achieved using a capacitive energy storage device of fixed capacitance, without the need for switching circuits to vary the capacitance to meet varying energy requirements. A further advantage is that it obviates the need for inductors in the energy supply circuitry, thereby avoiding the discharge of relatively large currents through inductors so that the resulting apparatus is relatively free of mechanical shock and therefore quieter, and also creates less electromagnetic interference to surrounding equipment.

A further advantage is to provide greater flexibility in determining the energy and pulse length

The irradiating device will generally comprise a flash lamp connected to the input such that the current i is discharged through the flash lamp.

The estimated voltage value $V_E$ is preferably determined by the control unit processing the demand values of output energy $E_D$ and output pulse length $t_D$ according to a pre-determined algorithm defined below.

The radiant energy from the flash lamp may be delivered to an output aperture of the irradiating device for presentation to a treatment site in use.

A pulse of incoherent radiation in the form of visible light or infrared radiation may thereby be produced.

The estimated value of input energy $E_E$ required to be input to the flash lamp is preferably proportional to the demand value of output energy $E_D$, the estimated value of input pulse length $t_E$ being taken to be substantially equal to the demand value of output pulse length $t_D$. Optionally, the estimated value of input pulse length $t_E$ may additionally include a dwell period corresponding to the rise time of current through the flash lamp.

Alternatively, the irradiating device may comprise a flash lamp connected to the input and further comprising a laser, the method including the step of optically pumping the laser with light from the flash lamp. Laser light from the laser may then be delivered to an output aperture for presentation to a treatment site in use.

Preferably the laser comprises a ruby laser, the flash lamp being placed in close proximity with the ruby material.

The estimated value of input energy $E_E$ may then be determined by the control unit according to a predetermined algorithm to comprise an estimated pumping energy Ep required to be input to the flash lamp during an estimated pumping period $t_P$ before lasing commences and an estimated input energy $E_L$ required to be input to the flash lamp so as to sustain operation of the laser during a subsequent output pulse length $t_O$.

The control unit may also determine the estimated value of input pulse length $t_E$ to be the sum of the estimated pumping period $t_P$ and the demand value of output pulse length $t_D$, the estimated pumping period $t_P$ being determined by means of a predetermined algorithm.

The method may include the step of measuring the current i, inputting to the control unit a measured current signal $i_M$ corresponding to the instantaneous amplitude of the current, integrating the measured current signal $i_M$ over a period commencing with the closing of the switching means to obtain a cumulative value, the control unit opening the switching means when the cumulative value is equal to a threshold value corresponding to the input energy $E_I$ received by the flash lamp being equal to the estimated value of input energy $E_E$.

An advantage of such a method is that the energy input to the flash lamp can be closely controlled relative to the calculated estimate value of input energy $E_E$. The use of the measured current to provide feed back improves the accuracy with which the energy delivered to the flash lamp can be controlled, and thereby improves the accuracy with which the output energy $E_O$ is controlled. In practice, the estimated value of input energy $E_E$ will be subject to systematic and statistical error and, particularly in medical applications, accurate control of the pulse energy is to be preferred to accurate control of pulse length.

Alternatively, where accurate control of pulse length is preferred, the method may include the step of measuring the elapsed time t, from the closure of the switching means, the control unit opening the switching means when the elapsed time t, is greater than or equal to the estimate value of input pulse length $t_E$.

The control unit is preferably an analogue computer. This is particularly advantageous in some medical environments where digital processing equipment may introduce unwanted interference or may be considered to be unduly susceptible to external interference.

Preferably the control unit is operable to receive continuously variable values of the demand value of output energy $E_D$ and the demand value of output pulse length $t_D$.

The user may thereby be able to continuously and accurately adjust pulse energy and pulse length, and to control each of these parameters independently of each other.

The method may optionally include the step of measuring the radiant energy directed to the output aperture, comparing the measurement of radiant energy with the demand value of output energy $E_D$, and actuating a safety cut out to prevent further operation of the irradiating device if the measured value exceeds the demand value by a predetermined safety margin.

Preferred embodiments of the present invention will now be described by way of example only and with reference to the accompanying drawings of which;

Figure 1 is a schematic diagram illustrating the supply of current to an irradiating device in accordance with the present invention;

Figure 2 is a schematic diagram of an irradiating device comprising a flash lamp;

Figure 3 is a schematic diagram illustrating an alternative arrangement for the supply of current to an irradiating device;

Figure 4 is a schematic circuit diagram showing the discharge of current through a flash lamp;

Figure 5 is a graphical illustration of the approximation of drive voltage to a rectangular pulse shape;
Figure 6 is a schematic representation of an alternative irradiating device comprising a flash lamp and a laser;
Figure 7 is a graphical representation of pulse shapes for an irradiating device comprising a flash lamp;
Figure 8 is a graphical representation of pulse shapes for an irradiating device comprising a flash lamp and laser; and
Figure 9 is an energy level diagram for a ruby laser.

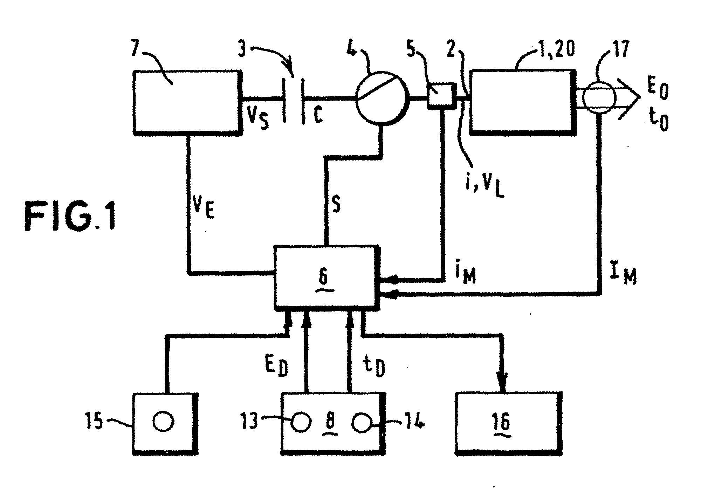

In Figure 1, an irradiating device 1 is required to deliver to a treatment site a pulse of light characterised by output energy $E_O$ and an output pulse length $t_O$. The irradiating device 1 has an input 2 which receives current i from a capacitive storage device 3 via a switching device 4. The capacitance storage device 3 comprises a bank of capacitors having a combined capacitance C.

A current sensor 5 comprising a transformer is arranged to sense the current i and output a measured current signal $i_M$ to a control unit 6. The switching device 4 is actuated by the control unit 6, an actuating signal S determining the opening and closing of the switching device 4.

The capacitive storage device 3 is charged to a supply voltage $V_S$ by means of a charging circuit 7 which is controlled by the control unit 6 such that the supply voltage $V_S$ is determined variably in accordance with an estimated voltage value $V_E$ produced by the control unit, the value of $V_S$ being proportional to $V_E$.

A user interface 8 in the form of a control panel allows a user to input a demand value of output energy $E_D$ and a demand value of output pulse length $t_D$ to the control unit 6, the demand values $E_D$ and $t_D$ corresponding to the values of radiant energy and pulse length required by the user to be delivered to the treatment site. The user interface 8 is provided with potentiometer controls 13 and 14 allowing the user to continuously vary the demand values $E_D$ and $t_D$.

A trigger 15 is also connected to the control unit 6, enabling the user to input an initiating signal to commence the actuation of the irradiating device 1.

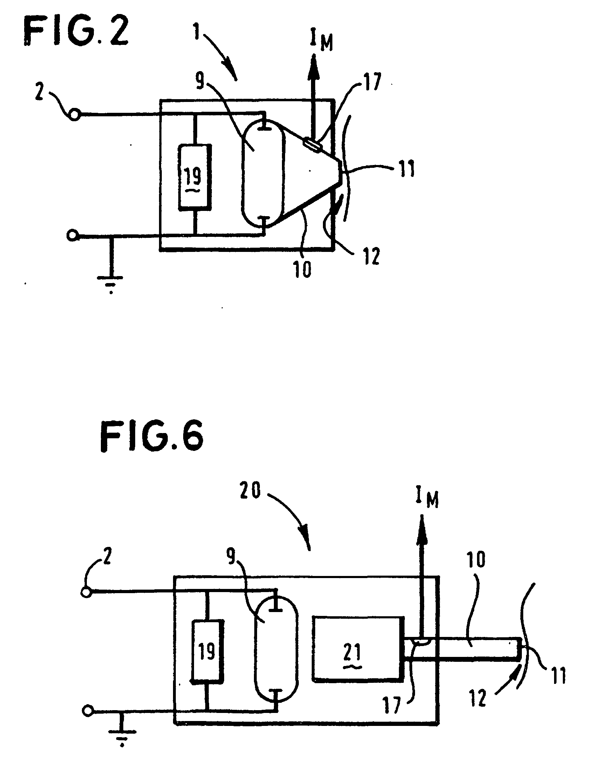

According to a first embodiment of the present invention, the irradiating device 1 as shown in Figure 2 consists of a flash lamp 9 in combination with a delivery device 10 arranged to collect and concentrate light emitted by the flash lamp and deliver the collected light to an output aperture 11. The output aperture 11 is comprises a window placed in contact with a localised area of skin at the treatment site 12.

The irradiating device 1 also includes a simmer circuit 19 which provides a relatively low level of continuous current through the flash lamp such that the flash lamp is maintained in a standby mode allowing the rapid onset of discharge in response to a voltage being applied via the switching device 4. Maintaining this low level of discharge avoids the need for a high voltage to initiate conduction by breakdown of the flash lamp gaseous medium.

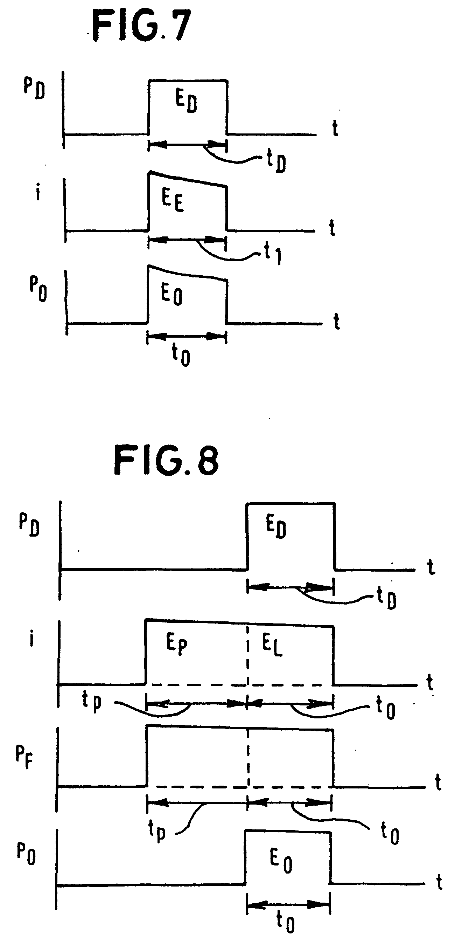

As illustrated in Figure 7 the demand value of energy $E_D$ translates to a demand value of power $P_D$ over the pulse length $t_D$, the pulse shape being ideally rectangular. The actual output power $P_O$ for an irradiating device 1 comprising a simple flash lamp 9 will vary proportionally to the current i passing through the flash lamp, the output pulse length $t_o$ corresponding to the input pulse length $t_i$ of current i input to the irradiating device. The level of current i passing through the flash lamp 9 will be determined by the supply voltage $V_S$, taking into account the impedance $k_O$ of the flash lamp, the impedance R of the switching device 4 and associated conductors. Referring to Figure 1, the control unit 6 comprises an analogue computer configured to determine the estimated voltage value $V_E$ which must be delivered to the charging circuit 7 in order to achieve the supply voltage $V_S$ appropriate to a user requirement defined by $E_D$, $t_D$. The charging circuit 7 is then operable to charge the capacitive storage device 3 until the required supply voltage $V_S$ is achieved, the "ready" status of the charging unit then being indicated to the user by means of an indicator panel 16. The user is then able to initiate irradiation by means of the trigger 15 in response to which the control unit 6 generates the actuating signal S to close the switching device 4. Current i then commences to flow through the flash lamp 9 as the capacitive storage device 3 progressively discharges. A measured current signal $i_M$ is input to the control unit 6 from current sensor 5 and the control unit performs an integration to determine a measure of input energy $E_M$ delivered to the flash lamp.

The control unit 6 also determines from the demand value of output energy $E_D$ an estimated value of input energy $E_E$ required to be supplied to the input 2 in order to produce a value of output energy $E_O$ equal to the demand value of output energy $E_D$. The measured value of input energy $E_M$ is compared with the estimated value of input energy $E_E$ and the actuating signal S is terminated to open the switching device 4 when $E_M = E_E$.

The relationship between $E_E$ and $E_D$ is established by calibration measurements of $E_O$ and the control unit 6 configured accordingly.

The resulting output pulse length $t_O$ of the light pulse delivered to the treatment site 12 will in general correspond closely to the demand value of output pulse length $t_D$. Some variation is however expected since for example the rise time of the current i will vary from pulse to pulse following closure of the switching device 4, this phenomenon being referred to typically as jitter of the flash lamp 9. The measured current signal $i_M$ thereby enables the control unit 6 to provide feed-back to accurately control the output energy $E_O$.

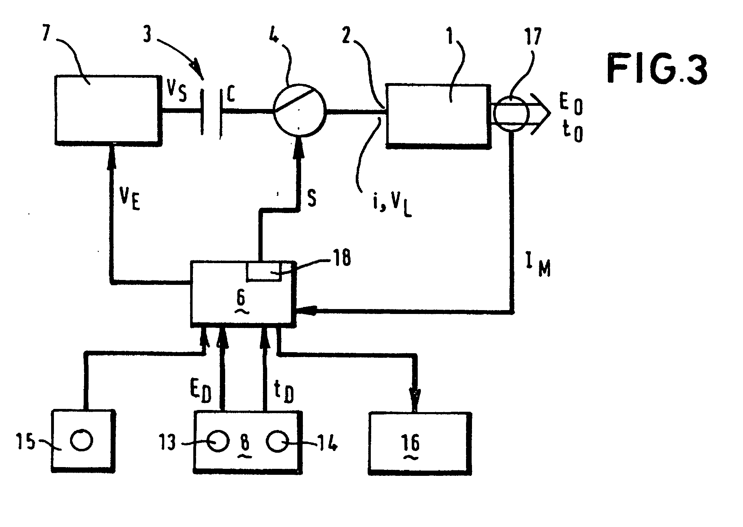

As illustrated in Figure 3, the shape of the pulse of current i is not exactly rectangular since the supply voltage

$V_S$ and hence current i will decay as the capacitive storage device 3 progressively discharges.

As a further safety feature, a light detector 17 is connected to the irradiating device 1 so as to input to the control unit 6 an intensity measurement signal $I_M$ which the control unit integrates to determine a measure of the output energy $E_O$ by means of a predetermined calibration constant. The control unit 6 compares the measurement of output energy $E_O$ with the demand value of output energy $E_D$ and indicates a fault condition if the difference exceeds a safety margin of 20%. In response to the fault condition being flagged, the control unit 6 ceases to respond to further actuation of the trigger 15.

The switching device 4 is an insulated gate bipolar transistor which is suited to the switching of high currents and enables the current i to be interrupted before the capacitive storage device 3 has been fully discharged, unlike current switching devices such as thyristors which require the current to fall to zero before the device turns off. In practice, the total energy stored in the capacitive storage device is between three and four times the maximum amount of energy required to be delivered at each pulse.

The control unit 6 calculates the estimated voltage value $V_E$ from the following algorithm.

$$V_E = V_T + \frac{E_E R}{t_E V_L} + V_L + \frac{E_E}{2CV_L}$$

where:

$$V_L = \sqrt[3]{\left(\frac{E_E k_0}{t_E}\right)}$$

$V_T$ = constant voltage across the switching device when conducting;
$k_O$ = input impedance of the flash lamp;
R = resistance between the capacitive storage device and the input of the irradiating device.

The manner in which this algorithm is derived is explained below at Appendix A.

The calculation according to the algorithm is performed by means of an analogue computer in the preferred embodiment but alternatively may be performed using an electronic digital processor such as a microcomputer running appropriate software.

A second embodiment shown in Figure 3 will now be described using corresponding reference numerals to those used above where appropriate for corresponding elements.

In the embodiment of Figure 3, the control unit 6 utilises a timing circuit 18 to generate the actuating signal S so as to precisely determine the timing of the closing and opening of the switching device 4 to correspond to the estimated value of input pulse length $t_E$. In this embodiment it is not necessary to measure the current i and so the transformer 5 has been omitted.

The control unit 6 continues to calculate $V_E$ according to the above algorithm. This simplified arrangement amounts to an open loop system in which no feed-back of input energy $E_I$ is utilised. Greater statistical fluctuation in the output energy $E_O$ is therefore anticipated in this embodiment but this embodiment may be preferred where it is considered more appropriate to have closer control over the output pulse length $t_O$.

A third embodiment will now be described using corresponding reference numerals to those of previous embodiments where appropriate for corresponding elements.

An alternative irradiating device 20 as shown in Figure 6 has an input 2 connected to a flash lamp 9 which is used to optically pump a ruby laser 21. The ruby laser 21 has a delivery device 10 in the form of an articulated arm arranged to deliver the coherent light output of the laser to an output aperture 11 which in use is deployed at a treatment site 12.

The schematic diagram of Figure 1 is also applicable to the embodiment of Figure 3 although the manner of operation of the control unit is different in an important respect. For each pulsed operation of the irradiating device 20, the flash lamp 9 is required not only to continue operation during the period during which the laser provides coherent light output, i.e. during the output pulse length $t_O$, but also during a pumping period $t_p$ during which the light output of the flash lamp excites the active material of the laser to a point where population inversion allows lasing to commence.

The control unit 6 will therefore determine an estimated value of input pulse length $t_E$ which exceeds the demand value of output pulse length $t_D$ by the pumping period $t_P$. Similarly, the control unit 6 will determine an estimated value of input energy $E_E$ which takes account of the additional energy required to be input to the flash lamp during the pumping period $t_P$.

Having determined the estimated value of input energy $E_E$ and the estimated value of input pulse length $t_E$, the above algorithm referred to with reference to the first and second embodiments is then implemented.

As shown schematically in Figure 9, the energy levels in a ruby laser may be visualised as comprising a ground state having population N1, a pumping level having a population N3, and a laser level having a population N2, the populations N1, N2 and N3 referring to electron energy populations in the active material. The pumping mechanism relies on the flash lamp providing excitation from the ground state to the pumping level from which population of the laser level is effected by decay from the pumping level with a relatively short time constant. Lasing at the frequency corresponding to the N2-N1 transition occurs when population inversion is achieved i.e. N2 > N1.

The pumping period $t_P$ during which the flash lamp will need to irradiate the active material of the laser before lasing commences will depend upon the time constant $\tau$ of the N2-N1 transition, the rate at which the flash lamp supplies energy to the active material and the ratio of N2 -N1 at which lasing occurs.

Given the demand values of energy and pulse length $E_D$, $t_D$, the control unit 6 is required to operate the flash lamp 9 for a sufficient period and with sufficient energy to achieve pumping of the laser and subsequently maintain laser action by re-populating the laser level N2. As illustrated in Figure 8, the control unit must determine an estimated value of input pulse length $t_E$ given by

$$t_E = t_P + t_D$$

where: $t_p$ is the pumping period and $t_D$ is the demand value of output pulse length which will be substantially equal to $t_o$, the actual output pulse length for which the laser output power $P_O$ is achieved.

The control unit may determine $t_P$ according to an algorithm of the following form;

$$t_P = \left[ -\frac{1}{n+1} \log_e \left\{ \left| \frac{n(1-x)-x}{n} \right| \right\} \right] \tau$$

where

$$n = \frac{\frac{E_L}{m} + x}{1-x}$$

and

$$m = \frac{t_D}{\tau}$$

where

$$x = \text{population inversion ratio} = \frac{N_2}{N_1 + N_2}$$

$\tau$ = lifetime of laser state
and
$E_L$ is a dimensionless parameter proportional to $E_D$ and representative of the energy supply by the flash lamp during lasing.

In order to determine the estimated voltage value $V_E$ the control unit 6 also requires the estimated value of input energy $E_E$ which may be computed according to the following algorithm;

$$E_E = B \frac{n}{\tau} (t_P + t_D)$$

where

B = a constant

In the above expressions the constants A and B may be determined empirically.

The control unit may therefore determine the required values of $t_E$ and $E_E$ from the user demand values of $t_D$ and $E_D$ and then control the flash lamp operation as indicated above with reference to the first embodiment.

The present invention has been described above primarily with reference to medical irradiating devices. The present invention may alternatively be utilised in the operation of irradiating devices for other purposes such as drilling and welding where both radiation pulse length and energy need to be accurately controlled. An irradiating device for drilling or welding may for example comprise a YAG laser pumped by a flash lamp, the flash lamp being connected to the input of the irradiating device so as to receive current from a capacitive energy storage device via a switching means as described above.

Appendix A

For a given pulse length and pulse energy into a flash lamp there is a unique drive voltage. However the ideal case is not easily achieved in reality as effects such as capacitor bank voltage droop during the pulse (due to the finite capacitor size), resistive cable voltage drop and constant drop due to bipolar switching devices reduce the actual voltage appearing across the lamp terminals. Compensation factors have to be added in to adjust the capacitor bank voltage to allow for these drops.

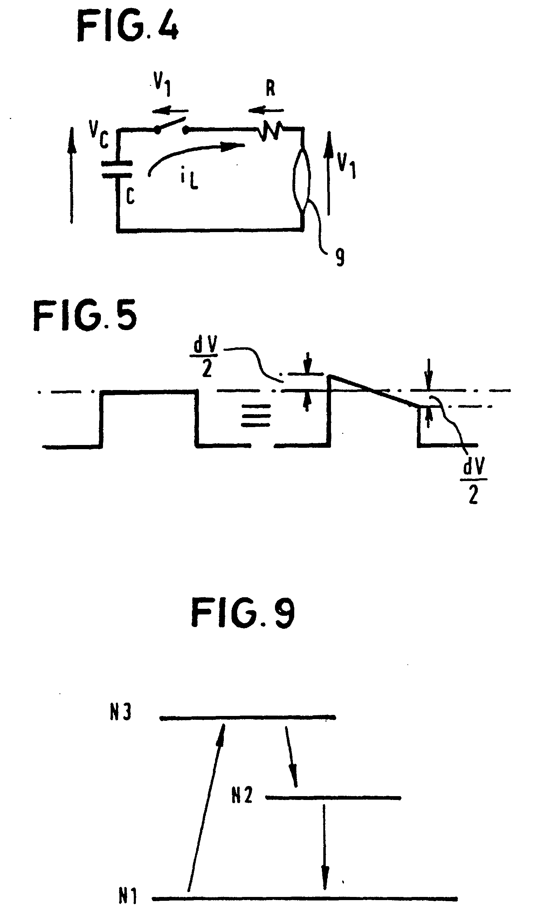

The proposed schematic is shown in Figure 4.

Ideally, the current through a flash lamp, at currents above the voltage minimum on the I-V curve, is related to the voltage and lamp impedance by:

$$V_L = k_O \sqrt{i} \qquad\qquad (1);$$

where:

$V_L$ =    Lamp Voltage;
$k_O$ =    Lamp impedance;
$i$ =    Lamp current;

For a constant voltage the energy in to the lamp is given by:

$$E_E = V_L . i . t_E \qquad\qquad (2);$$

where:

$E_E$ =    Estimated value of lamp energy required;
$t_E$ =    Estimated pulse length required;

The energy stored in the capacitor is given by:

$$E_C = \frac{1}{2} C V_C^2 \qquad\qquad (3);$$

where:

$E_C$ =    Energy stored in storage capacitor;
$C$ =    Storage capacitor;
$V_C$ =    Capacitor voltage.

To account for voltages across the wiring to the lamp and the voltage across the switching device, the estimated voltage value for charging the capacitor is given by:

$$V_E = V_T + i R + V_L \qquad (4);$$

where:

$V_T$ = Constant voltage across the switching device;
$R$ = Resistance of connections from capacitor to lamp.

If we combine and rearrange (1) and (2) we get:

$$V_L = \sqrt[3]{\left(\frac{E_L k_0^2}{t_1}\right)} \qquad (5);$$

Also from (1):

$$i = \frac{V_L^2}{k_O^2} \qquad (6);$$

Substituting (1) and (2) in (6) and using (5), (4) becomes:

$$V_E = V_T + \left(\frac{E_E}{t_E}\right)\frac{1}{V_L}R + \sqrt[3]{\left(\frac{E_E k_0^2}{t_E}\right)} \qquad (7);$$

A final compensation must be made to adjust for the finite size of the capacitor storage element. For practical reasons the total energy stored is optimum at 3 to 4 times the maximum required pulse energy. The above calculations are based upon a fixed drive voltage but in the practical situation this is not the case.
From (3), for a given change in capacitor voltage the change in stored energy is given by:

$$\frac{\delta E_c}{\delta V_c} = \frac{1}{2}C.2.V_c \qquad (8);$$

For simplicity we now make two approximations. Firstly that for small changes in drive voltage the lamp current varies linearly as shown in Figure 5. We can then say that if we increase the storage capacitor voltage by half the predicted voltage droop, then the total energy delivered to the lamp will be correct.
Secondly for true compensation we need to use the adjusted capacitor voltage $V_C$ in our calculation for voltage droop, this would however lead to an iterative calculation. Therefore $V_C$ is approximated to $V_L$. The final compensation function is:

$$V_E = V_T + \frac{E_E R}{t_E V_L} + V_L + \frac{E_E}{2C_{VL}}$$

where:

$$V_L = \sqrt[3]{\left(\frac{E_E k_0}{t_E}\right)} \cdot$$

## Claims

1. A method of operating an irradiating device (1, 9, 20) to provide a discharge therefrom in the form of a pulse ($E_D$, $t_D$) of radiant energy, the irradiating device (9) having an input (2) selectively coupled to a capacitive energy storage device (3) via a controllable switching means (4), the method **characterised by**:

   charging the capacitive energy storage device (3) to a voltage $V_S$, wherein a total stored energy in the capacitive energy storage device is between three and four times a maximum energy required to be delivered at each pulse;
   closing the switching means (4) to initiate a supply of current (i), associated with discharge of energy from the capacitive energy storage device, to the input (2) of the irradiating device (1, 9, 20) to cause generation of the pulse of radiant energy ($E_D$, $t_D$); and
   opening the switching means (4) to terminate the pulse of radiant energy ($E_D$, $t_D$) such that the supply of current to the input of the irradiating device (1, 9, 20) is terminated before complete discharge of the capacitive energy storage device (3).

2. The method of operating an irradiating device according to claim 1, wherein charging of the capacitive energy storage device further includes setting the voltage applied to the capacitive energy storage device to a value that represents a desired rate of energy into the irradiating device (1, 9, 20).

3. The method of operating an irradiating device according to claim 1 or 2, wherein the total stored energy in the capacitive energy storage device proportionally reflects a power to be delivered during a pulse of radiant energy from the irradiating device.

4. The method of operating an irradiating device according to claim 1, 2 or 3, wherein a pulse of radiant energy reduces an initial total stored energy of the capacitive energy storage device by no more than one third.

5. The method of operating an irradiating device according to any preceding claim, wherein a control unit is coupled to the switching means for operational control thereof, and the method further includes:

   inputting to the control unit a variable demand value of output energy ($E_D$) representative of a user requirement of energy to be discharged as a pulse of radiant energy from the irradiating device;
   inputting to the control unit a variable demand value of output pulse length ($t_D$) corresponding to a user requirement of pulse length of the pulse of radiant energy; and
   determining by operation of the control unit an estimated voltage value ($V_E$) representative of a voltage to which the energy storage device is required to be charged to enable the irradiating device to receive sufficient electrical current for the pulse of radiant energy to have actual values of output energy and output pulse length (Eo, to) corresponding substantially to the demand value of output energy ($E_D$) and the demand value of output pulse length ($t_D$) respectively; .
   and wherein the voltage is a supply voltage corresponding to the estimated voltage value.

6. The method of operating an irradiating device according to claim 5, wherein the estimated voltage value $V_E$ is determined by the control unit processing the demand values of output energy $E_D$ and output pulse length $t_D$ according to a pre-determined algorithm.

7. The method of operating an irradiating device according to claim 6, wherein the algorithm comprises:

$$V_E = V_T + \frac{E_E R}{t_E V_L} + V_L + \frac{E_E}{2C_{VL}}$$

$$V_L = \sqrt[3]{\left(\frac{E_E k_O}{t_E}\right)}$$

where:

$V_L$ = the voltage at the input to the flash lamp;
$V_T$ = a constant voltage across the switching device;
$K_O$ = the input impedance of the flash lamp;
$R$ = the resistance between the capacitive storage device and the input of the flash lamp;
$C$ = capacitance of the capacitive energy storage device;

$E_E$ is an estimated value of input energy required to be input to the flash lamp in order to provide output energy $E_O$ equal to the demand value of output energy $E_D$; and
$t_E$ is an estimated value of input pulse length required to produce an output pulse length to corresponding to the demand value of output pulse length $t_D$.

8. The method of operating an irradiating device according to claim 7, wherein the estimated value of input energy $E_E$ to be input to the flash lamp is determined by the control unit as being proportional to the demand value of output energy $E_D$ and wherein the estimated value of input pulse length $t_E$ is determined by the control unit to be substantially equal to the demand value of output pulse length $t_D$.

9. The method of operating an irradiating device according to claim 7 or 8, wherein the estimated value of input energy $E_E$ is determined according to a predetermined algorithm by the control unit to comprise an estimated pumping energy $E_P$ required to be input to the flash lamp during a pumping period before lasing commences and an estimated laser input energy $E_L$ required to be input to the flash lamp during a subsequent output pulse length $t_o$ during which lasing occurs.

10. The method of operating an irradiating device according to claim 9. wherein the estimated value of input pulse length $t_E$ is determined by the control unit to be the sum of the estimated pumping period $t_P$ and the demand value of output pulse length $t_D$ and wherein the estimated pumping period $t_P$ is determined by means of a predetermined algorithm.

11. The method of operating an irradiating device according to any of claims 7 to 10, including:

measuring the current i;
inputting to the control unit a measured current signal $i_M$ corresponding to an instantaneous amplitude of the current;
integrating the measured current signal $i_M$ over a period commencing with the closing of the switching means to obtain a cumulative value, and wherein the control unit opens the switching means when the cumulative value is equal to a threshold value corresponding to the input energy $E_I$ received by a flash lamp being equal to the estimated value of input energy $E_E$.

12. The method of operating an irradiating device according to any of claims 7 to 11, including measuring the elapsed time $t_I$ from the closure of the switching means, and wherein the control unit opens the switching means when the elapsed time $t_I$ is greater than or equal to the estimate value of input pulse length $t_E$.

13. The method of operating an irradiating device according to any of claims 7 to 12, including:

measuring radiant energy directed to an output aperture;

comparing the measurement of radiant energy with the demand value of output energy $E_D$; and
actuating a safety cut out to prevent further operation of the irradiating device if the measured value exceeds the demand value by a predetermined safety margin.

14. The method of operating an irradiating device according to any of claims 5 to 13, wherein the control unit is operable to receive continuously variable values of the demand value of output energy $E_D$ and the demand value of output pulse length $t_D$.

15. The method of operating an irradiating device according to any of claims 1 to 4, further comprising:

determining an amount of energy applied to the irradiating device;
setting a desired energy requirement for the pulse ($E_D$, $t_D$) of radiant energy; and
controlling the switching means to deliver the desired energy requirement in response to the determined amount of energy applied to the irradiating device.

16. The method of operating an irradiating device according to claim 15, further comprising:

measuring both current applied to the irradiating device (1, 9, 20) and a voltage across the irradiating device (1, 9, 20) to determine an amount of power applied to the irradiating device; and
determining an energy applied to the irradiating device by integrating the amount of power over time.

17. The method of operating an irradiating device according to any preceding claim, further including:

delivering radiant energy from a flash lamp to an output aperture of the irradiating device for presentation, in use, to a treatment site.

18. The method of operating an irradiating device according to any of claims 1 to 16, wherein the irradiating device comprises a flash lamp connected to the input and further comprising a laser, the method further comprising:

optically pumping the laser with light from the flash lamp and delivering laser light from the laser to an output aperture of the irradiating device for presentation, in use, to a treatment site.

19. The method of operating an irradiating device according to claim 18, wherein the laser is a ruby laser.

20. The method of operating an irradiating device according to any preceding claim, wherein the switching device comprises a gated semi-conductor device.

21. A method of operating an irradiating device as claimed in any preceding claim, including operating a flash lamp in a standby mode in which a relatively low level of continuous discharge is established through the flash lamp.

22. An irradiating system having an irradiating device (1, 9, 20) comprising an input (2) selectively coupled, via a controllable switch (4), to a capacitive energy storage device (3) connectable, in use, to a voltage source $V_S$, the controllable switch (4) operationally closeable to initiate a supply of current (i), associated with discharge of energy from the capacitive energy storage device (3), to the input (2) of the irradiating device (1, 9, 20) to cause generation of a pulse ($E_D$, $t_D$) of radiant energy having an energy up to a maximum energy; the irradiating system **characterised in that**:

the controllable switch (4) is operable to terminate the pulse of radiant energy by terminating the supply of current to the input (2) of the irradiating device (1, 9, 20) at a point before complete discharge of the capacitive energy storage device (3) and a total stored energy in the capacitive energy storage device is between three and four times the maximum energy required to be delivered at each pulse.

23. The irradiating system according to claim 22, wherein the irradiating device comprises a flash lamp (9) connected to the input (2) such that the current i is discharged through the flash lamp.

24. The irradiating system according to claim 22 or 23, further comprising:

means for selecting a voltage to be applied to the capacitive energy storage device, the voltage representative of a desired rate of energy transfer into the irradiating device (1, 9, 20).

**25.** The irradiating system according to claim 22, 23 or 24, wherein the total stored energy in the capacitive energy storage device reflects a power to be delivered during a pulse of radiant energy from the irradiating device.

**26.** The irradiating system according to any of claims 22 to 25, wherein a pulse of radiant energy is arranged to reduce an initial total stored energy of the capacitive energy storage device by no more than one third.

**27.** The irradiating system according to any of claims 22 to 26, further comprising:

means (5, 6) for determining an amount of energy applied to the irradiating device;
means (8) for setting a desired energy requirement for the pulse ($E_D$, $t_D$) of radiant energy; and
means (6) for controlling the switching means to deliver the desired energy requirement in response to the determined amount of energy applied to the irradiating device.

**28.** The irradiating system according to claim 22, wherein the controllable switch is coupled to a control unit arranged to actuate the controllable switch, the irradiating system further comprising:

means (13, 14) for inputting to the control unit (6) a variable demand value of output energy ($E_D$) representative of a user requirement of energy to be discharged as a pulse of radiant energy from the irradiating device;
means (13, 14) for inputting to the control unit a variable demand value of output pulse length ($t_D$) corresponding to a user requirement of pulse length of the pulse of radiant energy;
the control unit (6) being operable to control an estimated voltage value ($V_E$) representative of a voltage to which the capacitive energy storage device (3) is required to be charged to enable the irradiating device (1, 9, 20) to receive sufficient electrical current for the pulse of radiant energy to have actual values of output energy and output pulse length ($E_O$, $t_D$) corresponding substantially to the demand value of output energy ($E_D$) and the demand value of output pulse length ($t_D$) respectively;
a charging circuit (7) operable to charge the capacitive energy storage device (3) to a supply voltage Vs corresponding to the estimated voltage value;
a trigger (15) operable to close the controllable switch (4) to initiate a supply of current (i) to the input of the irradiating device (1, 9, 20); and

wherein the control unit (6) is operable to open the controllable switch to terminate the resulting pulse of radiant energy such that the supply of current to the input (2) of the irradiating device (1, 9, 20) is terminated before complete discharge of the energy storage device.

**Patentansprüche**

**1.** Verfahren zum Betreiben einer Strahlungseinrichtung (1, 9, 20), um eine Entladung der Strahlungseinrichtung in Form eines Strahlungsenergie-Impulses ($E_D$, $t_D$) herbeizuführen, wobei ein Eingang (2) der Strahlungseinrichtung (9) über eine steuerbare Schalteinrichtung (4) selektiv mit einer kapazitiven Energiespeichereinrichtung (3) gekoppelt wird, wobei das Verfahren **gekennzeichnet ist durch**:

- das Laden der kapazitiven Energiespeichereinrichtung (3) auf eine Spannung $V_s$, wobei die gesamte gespeicherte Energie in der kapazitiven Energiespeichereinrichtung zwischen dem Drei- und Vierfachen einer Maximalenergie liegt, die pro Impuls abgegeben werden muss;
- das Schließen der Schalteinrichtung (4), um in Verbindung mit einer Entladung von Energie aus der kapazitiven Energiespeichereinrichtung eine Zufuhr von Strom (i) zu dem Eingang (2) der Strahlungseinrichtung (1, 9, 20) zu beginnen, um die Erzeugung des Strahlungsenergie-Impulses ($E_D$, $t_D$) zu bewirken; und
- das Öffnen der Schalteinrichtung (4), um den Strahlungsenergie-Impuls ($E_D$, $t_D$) zu beenden, so dass die Stromzufuhr zu dem Eingang der Strahlungseinrichtung (1, 9, 20) vor einer vollständigen Entladung der kapazitiven Energiespeichereinrichtung (3) beendet wird.

**2.** Verfahren zum Betreiben einer Strahlungseinrichtung nach Anspruch 1, wobei das Laden der kapazitiven Energiespeichereinrichtung ferner das Einstellen der an die kapazitive Energiespeichereinrichtung angelegten Spannung auf einen Wert umfasst, der eine gewünschte Energieübertragungsrate zur Strahlungseinrichtung (1, 9, 20) repräsentiert.

EP 0 817 544 B1

3. Verfahren zum Betreiben einer Strahlungseinrichtung nach Anspruch 1 oder 2, wobei die gesamte gespeicherte Energie in der kapazitiven Energiespeichereinrichtung proportional eine Energie widerspiegelt, die während eines Strahlungsenergie-Impulses von der Strahlungseinrichtung abgegeben werden muss.

4. Verfahren zum Betreiben einer Strahlungseinrichtung nach Anspruch 1, 2 oder 3, wobei ein Strahlungsenergie-Impuls die zu Beginn insgesamt gespeicherte Energie der kapazitiven Energiespeichereinrichtung um nicht mehr als ein Drittel reduziert.

5. Verfahren zum Betreiben einer Strahlungseinrichtung nach einem der vorhergehenden Ansprüche, wobei zur Betriebssteuerung der Schalteinrichtung mit dieser Schalteinrichtung eine Steuereinrichtung gekoppelt ist und wobei das Verfahren ferner umfasst:

- das Eingeben eines variablen Ausgangsenergie-Bedarfswertes ($E_D$), der repräsentativ ist für die von einem Verbraucher geforderte Energie, die als ein Strahlungsenergie-Impuls von der Strahlungseinrichtung abzugeben ist, in die Steuereinheit;
- das Eingeben eines variablen Bedarfswertes für eine Ausgangsimpulsdauer ($t_D$) entsprechend einer von dem Verbraucher geforderten Impulsdauer des Strahlungsenergie-Impulses in die Steuereinheit; und
- die durch den Betrieb der Steuereinheit erfolgende Ermittlung eines voraussichtlichen Spannungswertes ($V_E$), der repräsentativ ist für eine Spannung, auf welche die Energiespeichereinheit geladen werden muss, um zu ermöglichen, dass die Strahlungseinrichtung elektrischen Strom erhält, der ausreicht, damit die tatsächlichen Werte von Ausgangsenergie und Ausgangsimpulsdauer ($E_O$, $t_O$) des Strahlungsenergie-Impulses jeweils im wesentlichen dem Bedarfswert für die Ausgangsenergie ($E_D$) und dem Bedarfswert für die Ausgangsimpulsdauer ($t_D$) entsprechen;
- und wobei die Spannung eine dem voraussichtlichen Spannungswert entsprechende Speisespannung ist.

6. Verfahren zum Betreiben einer Strahlungseinrichtung nach Anspruch 5, wobei der voraussichtliche Spannungswert $V_E$ durch die Steuereinheit ermittelt wird, die die Bedarfswerte für die Ausgangsenergie $E_D$ und für die Ausgangsimpulsdauer $t_D$ gemäß einem vorgegebenen Algorithmus verarbeitet.

7. Verfahren zum Betreiben einer Strahlungseinrichtung nach Anspruch 6, wobei der Algorithmus umfasst:

$$V_E = V_T + \frac{E_E R}{t_E V_L} + V_L + \frac{E_E}{2C_{VL}}$$

$$V_L = \sqrt[3]{\left(\frac{E_E k_O}{t_E}\right)}$$

dabei ist:

$V_L$ = die Spannung an dem Eingang zur Blitzlampe;
$V_T$ = eine konstante Spannung über der Schalteinrichtung;
$K_O$ = die Eingangsimpedanz der Blitzlampe;
$R$ = der Widerstand zwischen der kapazitiven Speichereinrichtung und dem Eingang der Blitzlampe;
$C$ = die Kapazität der kapazitiven Energiespeichereinrichtung;
$E_E$ = ein voraussichtlicher Wert der Eingangsenergie, die der Blitzlampe zugeführt werden muss, um eine Ausgangsenergie $E_O$ gleich dem Ausgangsenergie-Bedarfswert $E_D$ bereitzustellen; und
$t_E$ = ein voraussichtlicher Wert der Eingangsimpulsdauer, die notwendig ist, um eine dem Bedarfswert für die Ausgangsimpulsdauer $t_D$ entsprechende Ausgangsimpulsdauer $T_O$ zu erzeugen.

8. Verfahren zum Betreiben einer Strahlungseinrichtung nach Anspruch 7, wobei der voraussichtliche Wert der in die Blitzlampe einzuleitenden Eingangsenergie $E_E$ durch die Steuereinheit als ein zu dem Ausgangsenergie-Bedarfswert $E_D$ proportionaler Wert bestimmt wird und wobei der voraussichtliche Wert der Eingangsimpulsdauer $t_E$ durch die Steuereinheit so bestimmt wird, dass er im wesentlichen gleich dem Bedarfswert für die Ausgangsimpulsdauer $t_D$ ist.

13

9. Verfahren zum Betreiben einer Strahlungseinrichtung nach Anspruch 7 oder 8, wobei der voraussichtliche Wert der Eingangsenergie $E_E$ gemäß einem vorgegebenen Algorithmus durch die Steuereinheit derart bestimmt wird, dass er eine voraussichtliche Pumpenergie $E_P$, die während einer Pumpperiode vor dem Beginn der Lasertätigkeit der Blitzlampe zugeführt werden muss, und eine voraussichtliche Laser-Eingangsenergie $E_L$, die während einer anschließenden Ausgangsimpulsdauer $t_O$, während der die Lasertätigkeit stattfindet, der Blitzlampe zugeführt werden muss, umfasst.

10. Verfahren zum Betreiben einer Strahlungseinrichtung nach Anspruch 9, wobei der voraussichtliche Wert der Eingangsimpulsdauer $T_E$ durch die Steuereinheit derart bestimmt wird, dass dieser die Summe aus der voraussichtlichen Pumpperiode $t_P$ und dem Bedarfswert für die Ausgangsimpulsdauer $t_O$ ist, und wobei die voraussichtliche Pumpperiode $t_P$ mittels eines vorgegebenen Algorithmus bestimmt wird.

11. Verfahren zum Betreiben einer Strahlungseinrichtung nach einem der Ansprüche 7 bis 10, umfassend:

   - das Messen des Stroms i;
   - das Eingeben eines einer momentanen Amplitude des Stroms entsprechenden Messstromsignals iM in die Steuereinheit;
   - das Integrieren des Messstromsignals iM über eine mit dem Schließen der Schalteinrichtung beginnende Periode zur Gewinnung eines kumulativen Wertes, wobei die Steuereinheit die Schalteinrichtung öffnet, wenn der kumulative Wert gleich einem Schwellenwert ist, der der von einer Blitzlampe empfangenen Eingangsenergie $E_I$ entspricht, die gleich dem voraussichtlichen Wert der Eingangsenergie $E_E$ ist.

12. Verfahren zum Betreiben einer Strahlungseinrichtung nach einem der Ansprüche 7 bis 11, umfassend das Messen der ab dem Schließen der Schalteinrichtung verstrichenen Zeit $t_I$, wobei die Steuereinheit die Schalteinrichtung öffnet, wenn die verstrichene Zeit $t_I$ gleich oder größer ist als der voraussichtliche Wert der Eingangsimpulsdauer $t_E$.

13. Verfahren zum Betreiben einer Strahlungseinrichtung nach einem der Ansprüche 7 bis 12, umfassend:

   - das Messen von Strahlungsenergie, die auf eine Ausgangsöffnung gerichtet wird;
   - das Vergleichen der Messung von Strahlungsenergie mit dem Ausgangsenergie-Bedarfswert $E_D$; und
   - das Betätigen eines Sicherheitsausschalters, um einen weiteren Betrieb der Strahlungseinrichtung zu verhindern, wenn der Messwert den Bedarfswert um eine vorgegebene Sicherheitsspanne überschreitet.

14. Verfahren zum Betreiben einer Strahlungseinrichtung nach einem der Ansprüche 5 bis 13, wobei die Steuereinheit betreibbar ist, um kontinuierlich variable Werte des Bedarfswertes für die Ausgangsenergie $E_D$ und des Bedarfswertes für die Ausgangsimpulsdauer $t_D$ zu erhalten.

15. Verfahren zum Betreiben einer Strahlungseinrichtung nach einem der Ansprüche 1 bis 4, ferner umfassend:

   - das Ermitteln eines der Strahlungseinrichtung zugeführten Energiebetrags;
   - das Einstellen eines gewünschten Energiebedarfs für den Strahlungsenergie-Impuls ($E_D$, $t_D$); und
   - das Steuern der Schalteinrichtung, um den gewünschten Energiebedarf in Reaktion auf den ermittelten Betrag der der Strahlungseinrichtung zugeführten Energie bereitzustellen.

16. Verfahren zum Betreiben einer Strahlungseinrichtung nach Anspruch 15, ferner umfassend:

   - das Messen sowohl des an die Strahlungseinrichtung (1, 9, 20) angelegten Stroms als auch einer Spannung über der Strahlungseinrichtung (1, 9, 20), um einen an die Strahlungseinrichtung abgegebenen Leistungsbetrag zu ermitteln; und
   - das Bestimmen einer der Strahlungseinrichtung zugeführten Energie, indem der Leistungsbetrag über die Zeit integriert wird.

17. Verfahren zum Betreiben einer Strahlungseinrichtung nach einem der vorhergehenden Ansprüche, ferner umfassend:

   - das Liefern von Strahlungsenergie von einer Blitzlampe zu einer Ausgangsapertur der Strahlungseinrichtung, um die Strahlungsenergie bei Betrieb an einer Behandlungsstelle anzubieten.

18. Verfahren zum Betreiben einer Strahlungseinrichtung nach einem der Ansprüche 1 bis 16, wobei die Strahlungseinrichtung eine mit dem Eingang verbundene Blitzlampe und ferner einen Laser aufweist und wobei das Verfahren des weiteren umfasst:

- das optische Pumpen des Lasers mit Licht aus der Blitzlampe und das Liefern von Laserlicht aus dem Laser zu einer Ausgangsapertur der Strahlungseinrichtung, um das Laserlicht bei Betrieb an einer Behandlungsstelle anzubieten.

19. Verfahren zum Betreiben einer Strahlungseinrichtung nach Anspruch 18, wobei der Laser einer Rubinlaser ist.

20. Verfahren zum Betreiben einer Strahlungseinrichtung nach einem der vorhergehenden Ansprüche, wobei die Schalteinrichtung eine torgesteuerte Halbleitereinrichtung umfasst.

21. Verfahren zum Betreiben einer Strahlungseinrichtung nach einem der vorhergehenden Ansprüche, umfassend das Betreiben einer Blitzlampe in einem Standby-Modus, in dem durch die Blitzlampe ein relativ niedriges Niveau einer kontinuierlichen Entladung geschaffen wird.

22. Strahlungssystem mit einer Strahlungseinrichtung (1, 9, 20), umfassend einen Eingang (2), der über einen steuerbaren Schalter (4) selektiv mit einer bei Betrieb an eine Spannungsquelle $V_s$ anschließbaren kapazitiven Energiespeichereinrichtung (3) verbunden werden kann, wobei der steuerbare Schalter (4) operativ geschlossen werden kann, um in Verbindung mit der Entladung von Energie aus der kapazitiven Energiespeichereinrichtung (3) eine Zufuhr von Strom (i) zu dem Eingang (2) der Strahlungseinrichtung (1, 9, 20) zu beginnen, um die Erzeugung eines Strahlungsenergie-Impulses ($E_D$, $t_O$) mit einer Energie bis hin zu einer maximalen Energie zu bewirken; wobei das Strahlungssystem **dadurch gekennzeichnet ist,**
**dass** der steuerbare Schalter (4) betätigbar ist, um den Strahlungsenergie-Impuls durch die Beendigung der Stromzufuhr zu dem Eingang (2) der Strahlungseinrichtung (1, 9, 20) an einem Punkt vor einer vollständigen Entladung der kapazitiven Energiespeichereinrichtung zu beenden, und dass die gesamte gespeicherte Energie in der kapazitiven Energiespeichereinrichtung zwischen dem Drei- und Vierfachen der maximalen Energie liegt, die pro Impuls abgegeben werden muss.

23. Strahlungssystem nach Anspruch 22, wobei die Strahlungseinrichtung eine Blitzlampe (9) umfasst, die mit dem Eingang (2) derart verbunden ist, dass der Strom i durch die Blitzlampe abgegeben wird.

24. Strahlungssystem nach Anspruch 22 oder 23, ferner umfassend eine Einrichtung für die Wahl einer an die kapazitive Energiespeichereinrichtung anzulegenden Spannung, wobei die Spannung repräsentativ ist für eine gewünschte Energieübertragungsrate zur Strahlungseinrichtung (1, 9, 20).

25. Strahlungssystem nach Anspruch 22, 23 oder 24, wobei die gesamte gespeicherte Energie in der kapazitiven Energiespeichereinrichtung eine Leistung widerspiegelt, die während eines Strahlungsenergie-Impulses von der Strahlungseinrichtung erbracht werden muss.

26. Strahlungssystem nach einem der Ansprüche 22 bis 25, wobei ein Strahlungsenergie-Impuls derart vorgesehen ist, dass dieser eine zu Beginn insgesamt gespeicherte Energie der kapazitiven Energiespeichereinrichtung um nicht mehr als ein Drittel verringert.

27. Strahlungssystem nach einem der Ansprüche 22 bis 26, ferner umfassend:

- eine Einrichtung (5, 6) zur Ermittlung eines der Strahlungseinrichtung zugeführten Energiebetrages;
- eine Einrichtung (8) zur Einstellung eines gewünschten Energiebedarfs für den Strahlungsenergie-Impuls ($E_D$, $t_D$); und
- eine Einrichtung (6) zum Steuern der Schalteinrichtung, um den gewünschten Energiebedarf in Reaktion auf den ermittelten Betrag der der Strahlungseinrichtung zugeführten Energie bereitzustellen.

28. Strahlungssystem nach Anspruch 22, wobei der steuerbare Schalter mit einer für seine Betätigung angeordneten Steuereinheit gekoppelt ist, wobei das Strahlungssystem ferner umfasst:

- eine Einrichtung (13, 14) zur Eingabe eines variablen Ausgangsenergie-Bedarfswertes ($E_D$), der repräsentativ ist für die von einem Verbraucher geforderte Energie, die als ein Strahlungsenergie-Impuls von der Strahlungseinrichtung abzugeben ist, in die Steuereinheit (6);
- eine Einrichtung (13, 14) zur Eingabe eines variablen Bedarfswertes für die Ausgangsimpulsdauer ($t_D$) entsprechend der von einem Verbraucher geforderten Impulsdauer des Strahlungsenergie-Impulses in die Steuereinheit;
- wobei die Steuereinheit (6) für die Steuerung eines voraussichtlichen Spannungswertes ($V_E$) betreibbar ist, der repräsentativ ist für eine Spannung, auf welche die kapazitive Energiespeichereinrichtung (3) geladen werden muss, um zu ermöglichen, dass die Strahlungseinrichtung (1, 9, 20) elektrischen Strom erhält, der ausreicht, damit die tatsächlichen Werte der Ausgangsenergie und der Ausgangsimpulsdauer ($E_O$, $t_O$) des Strahlungsenergie-Impulses jeweils im wesentlichen dem Ausgangsenergie-Bedarfswert ($E_D$) und dem Bedarfswert für die Ausgangsimpulsdauer ($t_D$) entsprechen;
- einen Ladekreis (7), der betreibbar ist für das Laden der kapazitiven Energiespeichereinrichtung (3) auf eine dem voraussichtlichen Spannungswert entsprechende Speisespannung $V_s$;
- einen Trigger (15), der betreibbar ist für das Schließen des steuerbaren Schalters (4), um eine Zuleitung von Strom (i) zu dem Eingang der Strahlungseinrichtung (1, 9, 20) zu beginnen; und
- wobei die Steuereinheit (6) betreibbar ist zum Öffnen des steuerbaren Schalters, um den resultierenden Strahlungsenergie-Impuls zu beenden, so dass die Zufuhr von Strom zu dem Eingang (2) der Strahlungseinrichtung (1, 9, 20) vor einer vollständigen Entladung der Energiespeichereinrichtung beendet wird.

**Revendications**

1. Procédé de fonctionnement d'un dispositif d'irradiation ou insolation (1, 9, 20) pour produire à partir de celui-ci une décharge sous la forme d'une impulsion ($E_D$, $t_D$) d'énergie de rayonnement, le dispositif d'irradiation ou insolation (9) ayant une entrée (2) couplée sélectivement à un dispositif de stockage capacitif d'énergie (3), par des moyens de commutation (4) pouvant être commandés, le procédé étant **caractérisé par** :

   le chargement du dispositif de stockage capacitif d'énergie (3), à une tension $V_s$, dans lequel l'énergie stockée totale dans le dispositif de stockage capacitif d'énergie est compris entre trois et quatre fois l'énergie maximale nécessaire à fournir à chaque impulsion ;
   la fermeture des moyens de commutation (4) pour provoquer une fourniture de courant (i), associée à la décharge d'énergie depuis le dispositif de stockage capacitif d'énergie, à l'entrée (2) du dispositif d'irradiation (1, 9, 20) pour provoquer la génération de l'impulsion d'énergie de rayonnement ($E_D$, $t_D$) ; et
   l'ouverture des moyens de commutation (4) pour achever l'impulsion d'énergie de rayonnement ($E_D$, $t_D$), de manière que la fourniture de courant à l'entrée du dispositif d'irradiation (1, 9, 20) soit achevée avant la décharge complète du dispositif de stockage capacitif d'énergie (3).

2. Procédé de fonctionnement d'un dispositif d'irradiation ou insolation selon la revendication 1, dans lequel le chargement du dispositif de stockage capacitif d'énergie comprend le réglage de la tension appliquée au dispositif de stockage capacitif d'énergie à une valeur représentant un taux d'énergie souhaité dans le dispositif d'irradiation (1, 9, 20).

3. Procédé de fonctionnement d'un dispositif d'irradiation ou insolation selon la revendication 1 ou 2, dans lequel l'énergie stockée totale dans le dispositif de stockage capacitif d'énergie reflète proportionnellement la puissance à fournir durant une impulsion d'énergie de rayonnement depuis le dispositif d'irradiation.

4. Procédé de fonctionnement d'un dispositif d'irradiation ou insolation selon la revendication 1, 2 ou 3, dans lequel une impulsion d'énergie de rayonnement réduit l'énergie stockée totale initiale du dispositif de stockage capacitif d'énergie d'une valeur non supérieure à un tiers.

5. Procédé de fonctionnement d'un dispositif d'irradiation ou insolation selon l'une quelconque des revendications précédentes, dans lequel l'unité de commande est couplée aux moyens de commutation pour assurer leur commande opérationnelle, et le procédé comprend en outre :

   l'introduction à l'unité de commande d'une valeur de demande variable en énergie de sortie ($E_D$) représentative d'une demande utilisateur en énergie à décharger sous forme d'impulsion d'énergie de rayonnement depuis le dispositif d'irradiation.
   l'introduction à l'unité de commande d'une valeur de demande variable de longueur d'impulsion de sortie ($t_D$) correspondant à une demande utilisateur de longueur d'impulsion concernant l'impulsion d'énergie de rayonnement ; et

la détermination, par le fonctionnement de l'unité de commande, d'une valeur de tension ($V_E$) estimée, représentative d'une tension à laquelle le dispositif de stockage d'énergie doit être chargé pour permettre au dispositif d'irradiation de recevoir suffisamment de courant électrique pour que l'impulsion d'énergie de rayonnement ait des valeurs réelles d'énergie produite et de longueur d'impulsion produite ($E_O$, $t_O$) correspondant sensiblement à la valeur demandée d'énergie de sortie ($E_D$) et à la valeur demandée de longueur d'impulsion de sortie ($t_D$) respectivement ;

et dans lequel la tension est une tension d'alimentation correspondant à la valeur de tension estimée.

6. Procédé de fonctionnement d'un dispositif d'irradiation ou insolation selon la revendication 5, dans lequel la valeur de tension $V_E$ estimée est déterminée par l'unité de commande traitant les valeurs de demande en énergie de sortie $E_D$ et en longueur d'impulsion de sortie $t_D$ , selon un algorithme prédéterminé.

7. Procédé de fonctionnement d'un dispositif de rayonnement selon la revendication 6, dans lequel l'algorithme comprend les équations suivantes :

$$V_E = V_T + \frac{E_E R}{t_E V_L} + V_L + \frac{E_E}{2C_{VL}}$$

$$V_L = 3\sqrt{\left(\frac{E_E k o}{t_E}\right)}$$

dans lesquelles :

$V_L$ =    tension à l'entrée de la lampe à éclat ;
$V_T$ =    tension constante dans le dispositif de commutation ;
$K_O$ =    impédance d'entrée de la lampe à éclat
$R$ =    résistance entre le dispositif de stockage capacitif et l'entrée de la lampe à éclat
$C$ =    capacité du dispositif de stockage capacitif d'énergie ;
$E_E$ =    est une valeur estimée de l'énergie d'entrée devant être introduite dans la lampe à éclat pour fournir l'énergie de sortie $E_O$ égale à la valeur demandée en énergie de sortie $E_D$ ; et
$T_E$    est une valeur estimée de la longueur d'impulsion d'entrée nécessaire pour produire une longueur d'impulsion de sortie $t_O$ correspondant à la valeur demandée de la longueur d'impulsion de sortie $t_D$.

8. Procédé de fonctionnement d'un dispositif de rayonnement selon la revendication 7, dans lequel la valeur estimée d'énergie d'entrée $E_E$ à introduire à la lampe à éclat est déterminée, par l'unité de commande, comme étant proportionnelle à la valeur demandée d'énergie de sortie $E_D$ et dans lequel la valeur estimée de la longueur d'impulsion d'entrée $t_E$ est déterminée par l'unité de commande comme étant sensiblement égale à la valeur demandée de la longueur d'impulsion de sortie $t_D$.

9. Procédé de fonctionnement d'un dispositif de rayonnement selon la revendication 7 ou 8, dans lequel la valeur estimée d'énergie d'entrée $E_E$ est déterminée selon un algorithme, prédéterminé par l'unité de commande, pour comprendre une énergie de pompage $E_P$ estimée, devant être introduite dans la lampe à éclat pendant une période de pompage précédant le fonctionnement en laser, et une énergie d'entrée laser estimée $E_L$ , devant être entrée dans la lampe à éclat durant une longueur d'impulsion de sortie $t_O$ subséquente, pendant que l'effet laser se produit.

10. Procédé de fonctionnement d'un dispositif de rayonnement selon la revendication 9, dans lequel la valeur estimée de la longueur d'impulsion d'entrée $t_E$ est déterminée par l'unité de commande pour être la somme de la période de pompage estimée $t_P$ et de la valeur demandée de longueur d'impulsion de sortie $t_D$ et dans lequel la période de pompage estimée $t_P$ est déterminée à l'aide d'un algorithme prédéterminé.

11. Procédé de fonctionnement d'un dispositif de rayonnement selon l'une quelconque des revendications 7 à 10, comprenant :

la mesure du courant i ;

l'entrée à l'unité de commande d'un signal de courant mesuré $i_M$ correspondant à une amplitude instantanée du courant ;

l'intégration du signal de courant mesuré $i_M$, sur une période commençant par la fermeture des moyens de commutation pour obtenir une valeur cumulative et dans lequel l'unité de commande ouvre les moyens de commutation lorsque la valeur cumulative est égale à une valeur seuil correspondant à l'énergie d'entrée $E_i$ reçue par une lampe à éclat, comme étant égale à la valeur estimée de l'énergie d'entrée $E_E$.

12. Procédé de fonctionnement d'un dispositif de rayonnement selon l'une quelconque des revendications 7 à 11, comprenant la mesure du temps écoulé $t_I$ depuis la fermeture des moyens de commutation et dans lequel l'unité de commande ouvre les moyens de commutation lorsque le temps écoulé $t_I$ est supérieur ou égal à la valeur estimée de la longueur d'impulsion d'entrée $t_E$.

13. Procédé de fonctionnement d'un dispositif de rayonnement selon l'une quelconque des revendications 7 à 12, comprenant :

la mesure de l'énergie de rayonnement dirigée sur une ouverture de sortie ;
la comparaison de la mesure d'énergie de rayonnement à la valeur demandée d'énergie de sortie $E_D$ ; et
l'actionnement d'un système de coupure de sécurité, devant empêcher la continuation du fonctionnement du dispositif d'irradiation si la valeur mesurée est supérieure à la valeur demandée, ceci d'une marge de sécurité prédéterminée.

14. Procédé de fonctionnement d'un dispositif de rayonnement selon l'une quelconque des revendications 5 à 13, dans lequel l'unité de commande est susceptible de fonctionner pour recevoir des valeurs, variables de façon continue, de la valeur demandée en énergie de sortie $E_D$ et de la valeur demandée de longueur d'impulsion de sortie $t_D$.

15. Procédé de fonctionnement d'un dispositif de rayonnement selon l'une quelconque des revendications 1 à 4, comprenant en outre :

la détermination d'une quantité d'énergie fournie au dispositif d'irradiation ;
la fixation d'une exigence d'énergie souhaitée pour l'impulsion ($E_D$, $t_D$), en énergie de rayonnement ; et
la commande des moyens de commutation pour satisfaire à une exigence d'une énergie souhaitée, en réponse à la quantité d'énergie déterminée appliquée au dispositif d'irradiation.

16. Procédé de fonctionnement d'un dispositif de rayonnement selon la revendication 15, comprenant en outre :

la mesure à la fois du courant appliqué au dispositif d'irradiation (1, 9, 20) et d'une tension dans le dispositif d'irradiation (1, 9, 20), afin de déterminer la puissance appliquée au dispositif d'irradiation ; et
la détermination d'énergie appliquée au dispositif d'irradiation par intégration de la quantité de puissance en fonction du temps.

17. Procédé de fonctionnement d'un dispositif de rayonnement selon l'une quelconque des revendications précédentes, comprenant en outre :

la fourniture d'énergie de rayonnement depuis une lampe à éclat à une ouverture de sortie du dispositif d'irradiation pour présentation, lors de son utilisation, à un site de traitement.

18. Procédé de fonctionnement d'un dispositif de rayonnement selon l'une quelconque des revendications 1 à 16, dans lequel le dispositif d'irradiation comprend une lampe à éclat reliée à l'entrée et comprenant en outre un laser, le procédé comprenant en outre :

le pompage optique du laser avec une lumière provenant de la lampe à éclat et la fourniture de la lumière laser du laser à une ouverture de sortie du dispositif d'irradiation, pour présentation en utilisation à un site de traitement.

19. Procédé de fonctionnement d'un dispositif d'irradiation selon la revendication 18, dans lequel le laser est un laser à rubis.

**20.** Procédé de fonctionnement d'un dispositif d'irradiation selon l'une quelconque des revendications, dans lequel le dispositif de commutation comprend un dispositif à semi conducteur à commande par grille.

**21.** Procédé de fonctionnement d'un dispositif d'irradiation selon l'une quelconque des revendications,. comprenant le fonctionnement d'une lampe à éclat en mode d'attente, dans lequel un niveau relativement faible de décharge continue est établi par la lampe à éclat.

**22.** Un système d'irradiation comportant un dispositif d'irradiation (1, 9, 20) comprenant une entrée (2) couplée sélectivement, via un interrupteur (4) pouvant être commandé, un dispositif de stockage capacitif d'énergie (3) pouvant en utilisation être connecté à une source de tension $V_s$, l'interrupteur (4) pouvant être commandé pouvant être fermé fonctionnellement pour lancer la fourniture de courant (i), associé à la décharge d'énergie depuis le dispositif de stockage capacitif d'énergie (3), à l'entrée (2) du dispositif d'irradiation (1, 9, 20), pour provoquer la génération d'une impulsion ($E_D$, $t_D$) d'énergie de rayonnement ayant une énergie allant jusqu'à une énergie maximale ; le système d'irradiation étant **caractérisé en ce que** :

l'interrupteur (4) susceptible d'être commandé peut fonctionner pour achever l'impulsion d'énergie de rayonnement en cessant la fourniture de courant à l'entrée (2) du dispositif de rayonnement (1, 9, 20) à un moment précédent la décharge complète du dispositif de stockage capacitif d'énergie (3), et l'énergie stockée totale dans le dispositif de stockage capacitif d'énergie est comprise entre trois et quatre fois l'énergie maximale devant être fournie à chaque impulsion.

**23.** Le système d'irradiation selon la revendication 22, dans lequel le dispositif d'irradiation comprend une lampe à éclat (9) reliée à l'entrée (2), de manière que le courant (i) soit déchargé par la lampe à éclat.

**24.** Le système d'irradiation selon la revendication 22 ou 23, comprenant en outre :

des moyens pour sélectionner une tension à appliquer au dispositif de stockage capacitif d'énergie, la tension étant représentative d'un taux de transfert d'énergie souhaité dans le dispositif d'irradiation (1, 9, 20).

**25.** Le système d'irradiation selon la revendication 22, 23 ou 24, dans lequel l'énergie stockée totale dans le dispositif de stockage capacitif d'énergie reflète une puissance à fournir durant une impulsion d'énergie de rayonnement provenant du dispositif d'irradiation.

**26.** Le système d'irradiation selon l'une quelconque des revendications 22 à 25, dans lequel une impulsion d'énergie de rayonnement est agencée pour réduire l'énergie stockée totale initiale du dispositif de stockage capacitif d'énergie d'une valeur non supérieure à un tiers.

**27.** Le système d'irradiation selon l'une quelconque des revendications 22 à 26, comprenant en outre :

des moyens (5, 6) pour déterminer une quantité d'énergie appliquée au dispositif d'irradiation ;
des moyens (8) pour fixer une exigence d'énergie souhaitée concernant l'impulsion ($E_D$, $t_D$) d'énergie de rayonnement ; et
des moyens (6) pour commander les moyens de commutation pour satisfaire à l'exigence d'énergie souhaitée, en réponse à la quantité d'énergie déterminée appliquée au dispositif d'irradiation.

**28.** Le système d'irradiation selon la revendication 22, dans lequel l'interrupteur pouvant être commandé est couplé à une unité de commande, agencée pour actionner l'interrupteur pouvant être commandé, le système d'irradiation comprenant en outre :

des moyens (13, 14) pour introduire dans l'unité de commande (6) une valeur de demande variable en énergie de sortie ($E_D$) représentative d'une demande utilisateur en énergie à décharger sous forme d'impulsion d'énergie de rayonnement depuis le dispositif d'irradiation.
des moyens (13, 14) pour introduire dans l'unité de commande une valeur de demande variable de longueur d'impulsion de sortie ($t_D$) correspondant à une exigence utilisateur concernant la longueur d'impulsion de l'énergie de rayonnement ;
l'unité de commande (6) étant susceptible de fonctionner pour commander une valeur de tension ($V_E$) estimée, représentative d'une tension à laquelle le dispositif de stockage capacitif d'énergie (3) doit être chargé, pour permettre au dispositif d'irradiation (1, 9, 20) de recevoir suffisamment de courant électrique pour que l'impulsion d'énergie de rayonnement ait des valeurs réelles d'énergie de sortie et de longueur d'impulsion de sortie ($E_O$, $t_O$) correspondant sensiblement à la valeur demandée en énergie de sortie ($E_D$) et à la valeur demandée de longueur d'impulsion de sortie ($t_D$), respectivement ;

un circuit de chargement (7) susceptible de fonctionner pour charger le dispositif de stockage capacitif d'énergie (3) à une tension d'alimentation $V_s$ correspondant à la valeur de tension estimée ;

un déclencheur (15) susceptible de fonctionner pour fermer l'interrupteur (4) pouvant être commandé afin de lancer la fourniture de courant (i) à l'entrée du dispositif d'irradiation (1, 9, 20) ; et

dans lequel l'unité de commande (6) est susceptible de fonctionner pour ouvrir l'interrupteur susceptible d'être commandé pour faire cesser l'impulsion résultante d'énergie de rayonnement, de manière que la fourniture de courant à l'entrée (2) du dispositif d'irradiation (1, 9, 20) s'achève avant la décharge complète du dispositif de stockage d'énergie.

FIG.1

EP 0 817 544 B1

# FIG.2

# FIG.6

# FIG.3

# FIG.4

# FIG.5

# FIG.9

# FIG.7

# FIG.8